# EUROPEAN PATENT SPECIFICATION

(11) **EP 3 707 235 B1**
(45) Date of publication and mention of the grant of the patent: **06.07.2022**
(21) Application number: 18799735.8
(22) Date of filing: 06.11.2018
(51) Int. Cl.: C12M 1/42, C12M 1/34, C12M 1/00

(54) **CELL COMPRESSION DEVICE**
ZELLKOMPRESSIONSGERÄT
DISPOSITIF DE COMPRESSION CELLULAIRE

(30) Priority: 06.11.2017 EP 17200056
(43) Date of publication of application: 16.09.2020
(73) Proprietor: Fondazione Istituto Firc Di Oncologia Molecolare (IFOM), 20139 Milano (IT)
(72) Inventor: LI, Qingsen, 20139 Milano (MI) (IT)
(74) Representative: Capasso, Olga
(86) International application number: PCT/EP2018/080304
(87) International publication number: WO 2019/086702

(56) References cited:
- WO-A1-01/68800
- US-A1- 2012 276 622
- US-A1- 2017 009 207

## Description

### TECHNICAL FIELD

The present invention relates to a cell compression device for applying dynamic compression stress to cells. The invention also relates to a kit for cell compression comprising a cell compression device and a mounting tool. In addition, the invention relates to a method for applying dynamic compression stress to cells.

### BACKGROUND

Cells are constantly exposed to wide range of mechanical stimuli (such as stretching and/or compression) in various organ movement, development and functions. These mechanical stimuli are essential to proper cell functions, such as for example migration, proliferation, differentiation, ... [Tse, Cheng et al. "Mechanical compression drives cancer cells toward invasive phenotype" Proc Natl Acad Sci U S A 109(3) : 911-6; Engler, Sen et al. "Matrix Elasticity Directs Stem Cell Lineage Specification" Cell 126(4): 677-689 (2006); Vogel and Sheetz "Local force and geometry sensing regulate cell functions" Nat Rev Mol Cell Biol 7(4): 265-275 (2006); Wozniak and Chen "Mechanotransduction in development: a growing role for contractility" Nat Rev Mol Cell Biol 10(1): 34-43 (2009)]. As confirmed in literature [Tse, Cheng et al. "Mechanical compression drives cancer cells toward invasive phenotype" Proc Natl Acad Sci U S A 109(3): 911-6; Wang, Tytell et al. "Mechanotransduction at a distance: mechanically coupling the extracellular matrix with the nucleus" Nat Rev Mol Cell Biol 10(1): 75-82. (2009); Shivashankar "Mechanosignaling to the cell nucleus and gene regulation" Annu Rev Biophys 40: 361-78 (2011)], cells sense such mechanical stimuli through a process called mechanotransduction. Abnormal mechanotransduction leads to several patho-genesis, such as cancer, asthma, or heart disease [Tse, Cheng et al. "Mechanical compression drives cancer cells toward invasive phenotype" Proc Natl Acad Sci U S A 109(3): 911-6; Jaalouk and Lammerding "Mechanotransduction gone awry" Nat Rev Mol Cell Biol 10(1): 63-73 (2009); DuFort, Paszek et al. "Balancing forces: architectural control of mechanotransduction" Nat Rev Mol Cell Biol 12(5): 308-19 (2011)]. In Mechanobiology studies, it is vital to advance technologies for the application of physiologically mimicking mechanical force to cultured cells and tissue due to the complex in vivo biological system.

In particular, cell compression devices have been developed by applying a weighted piston on cells, these devices showing that mechanical compression drives cancer cells towards invasive phenotype [Tse, Cheng et al. "Mechanical compression drives cancer cells toward invasive phenotype" Proc Natl Acad Sci U S A 109(3): 911-6]. The other compression technique made use of microfabricated PDMS spacers to define a vertical confinement to cells, which induces fast Amoeboid migration of slow mesenchymal cells [Liu, Le Berre et al. "Confinement and low adhesion induce fast amoeboid migration of slow mesenchymal cells" Cell 160(4): 659-72]. US 20110076758A1 describes another cell compression device using pressurized fluid to elastically deform a membrane and move pressurizing member downwardly to compress cells.

US 2017/009207 A1 discloses a cell compression device comprising a main body having a central internal cavity with a symmetry longitudinal axis and a lower opening aligned with said axis. Further, a cell layer, which comprises a flexible disk or membrane on or in which the cells are disposed, is pressurised by a moving piston plate that applies pressure onto the cell layer. Also foreseen are clamping means to clamp the membrane to the main body.

However, all the reported cell compression techniques available are limited in precision and dynamic control of force.

It is therefore an object of the present invention to provide a device that solves, at least in part, the above mentioned problems. In particular, to provide a cell compression device that is efficient, simple to use (compatible with high resolution live cell imaging) and able to perform high precision dynamic compression stress.

This object is achieved by the cell compression device, the kit and the method according to the independent claims. Further advantageous combinations and designs are given in the dependent claims therefrom.

### DESCRIPTION OF THE INVENTION

The cell compression device according to the present invention comprises a main body having a central internal cavity with a symmetry longitudinal axis A and a lower opening aligned with said axis A. Also, the device comprises a deformable membrane having a piston attached thereto, wherein the piston comprises a main portion with a symmetry longitudinal axis B and clamping means to clamp the membrane to the main body at the lower opening to close the central internal cavity so that the closed central internal cavity forms a variable pressure chamber, the attached piston being located outside said variable pressure chamber and the membrane being clamped to the main body in correspondence of clamping points, characterized in that in an assembled configuration, when the deformable membrane is clamped to the main body, said membrane is in a relaxed configuration, wherein the distance of two opposing clamping points is lower than the length of the membrane between said two opposing clamping points, and the symmetry longitudinal axis B of the main portion of the piston coincides with the symmetry longitudinal axis A of the central internal cavity.

Specifically, in an assembled configuration, that is when the deformable membrane is clamped to the main body, the membrane is in the relaxed configuration, the distance of two opposing clamping points being lower than the length of the membrane between said to opposing clamping points, and the symmetry longitudinal axis B of the main portion of the piston coincides with the symmetry longitudinal axis A of the central cavity.

Differently from prior art compression devices that mechanically move a pressurizing member to compress cells, the cell compression device according to the present application is based on the direct transfer of precise air pressure to piston and then cells through a relaxed or suspended thin membrane. In this way, it is possible to reach a compression to single cells at the resolution of nano newtons. Also, the cell compression device according to the present application can apply precise and computer program controlled dynamic compressive load to cells growing in any cell culture dish. The piston, that is in direct contact with cells, can be made from variety of materials and can apply a uniform or localized compressive load (indentation) to cells. In particular, the device can be adapted to any cell culture dish and can be compatible with any inverted microscope for high resolution real time live cell imaging.

The main body can have any suitable form to comprise a central internal cavity with a symmetry longitudinal axis. Preferably, the main body has a cylindrical or ring form and the central internal cavity can assume in turn a cylindrical or ring form aligned with the main body. In other words, the main body and the central internal cavity can be represented by two coaxial elements. Furthermore, the central internal cavity, has a lower opening that is in turn aligned with the symmetry longitudinal axis of the cavity and then with the main body. Preferably, the lower opening has a circular form.

The membrane is made of a deformable or resilient material such as silicone and can have any suitable form. Preferably, the membrane is a surface having a center of symmetry, such as a square-shaped surface, a circular-shaped surface, or the like. The piston can be represented by any element able to press on the cells and is attached to the membrane through appropriate connecting means, such as plasma treat bonding (for PDMS) or glue or the like. Alternatively, the piston can be integrated in the membrane so that piston and membrane can form a single structure. The piston is preferably attached at the center of symmetry of the surface of the membrane. In particular, the piston comprises a main portion having a symmetric shape and therefore having its own symmetry longitudinal axis. Preferably, the piston has the form of a cylinder or of a cube.

The deformability of the membrane allows the membrane to assume different configurations. According to the present application, it is intended that the membrane assumes a relaxed or suspended configuration when it is not under tension. In the present case, maintaining the membrane in a relaxed configuration after the clamping to the main body means that the distance between two clamping points on the main body is always lower than the length of the membrane between these two clamping points, wherein a clamping point is intended as the point where the membrane is in contact (and then clamped to) the main body. Accordingly, the membrane results in a suspended configuration, that is without tension, between these two points. With the expression "two opposing clamping points", it is intended two points - where the membrane is clamped to the main body - located at the maximum distance one from the other. In case for example the clamping points are distributed on a circular clamping region, the distance of two opposing clamping points represents the diameter of this circular clamping region.

The clamping means is any suitable means for fixing the membrane to the main body and specifically to a lower portion of the main body. Preferably, the clamping means comprises at least a projection element to be inserted in a recess element present in the main body. In a preferential embodiment, the clamping means has a ring shape having an upper edge acting as projecting element whereas the lower portion of the main body comprises a continuous annulus-shaped recess to receive the edge of the ring (clamping means) . In a clamping configuration, the membrane is placed and fixed at clamping points between the recess element and the projecting element.

It is noted that clamping the membrane to the lower portion of the main body at the lower opening determines the closure of the central internal cavity. Preferably, the closure is airtight. This means that the closed central internal cavity can function as a chamber in which the internal pressure can be varied by means of appropriate devices, such as a pressure regulator. As the membrane is relaxed/suspended, the increase in applied air pressure is not used to tense and move the elastic membrane (as in prior art), but is directly counter balanced by the compressed cells underneath. In this dynamic balanced way, it is possible to precisely and dynamically transfer air pressure to compress cells.

It is furthermore noted that clamping the membrane to the lower portion of the main body at the lower opening determines an automatic alignment of the piston with the center of the variable pressure chamber. In fact, the symmetry longitudinal axis B of the main portion of the piston coincides with the symmetry longitudinal axis A of the central internal cavity.

In order to vary the internal pressure of the closed central internal cavity (or variable pressure chamber), the central internal cavity is connectable to pressure regulator means. In particular, the pressure regulator means can comprise a pump and/or one or more valves to finely regulate the air pressure inside the chamber. Preferably, the pressure regulator means can determine a pressure variation between 0 mbar to 25 mbar.

The pressure regulator means is connected to the cell compression device through suitable pressure connecting means, such as a flexible tube or a hose. Accordingly, the central internal cavity is provided with a dedicated opening couplable to said connecting means.

According to one embodiment of the present application, the main body further comprises connection means to directly interlock the device to a cell culture dish. In particular, said connection means are located in the lower portion of the main body. Preferably, the connection means comprises mechanical ribs that abut against the internal edges of a Petri dish when the cell compression device is interlocked to the dish. In this way, the cell compression device is firmly fixed relative to the dish and the piston is in the position to carry out a load to the cells distributed on the (internal) bottom of the dish.

In particular, the device can have a circular shape that can be scaled down a diameter around 3.5 cm. In this way, the device according to the present invention is reduceable to the size of a cell culture dish. In particular, the device can be configured to be interlocked with a 3.5 cm cell culture Petri dish. Of course, the device can also be configured to be interlocked with cell culture dishes or culture wells of different diameters.

It is preferable that all the components of the main body are made of a plastic or other material suitable to be printed by means of a 3D-printer. This implies that the device can be mass produced easily by other industrial process and using different material.

The components of the device can also be made of different materials which are not necessary apt to be printed with a 3D printer but are suitable for the purpose of the present intention.

In a further embodiment of the present application, the piston can comprise a plurality of micropillars to apply localized compression (indentation) to the nucleus of the cells. The micropillars can be located at the bottom of the main portion of the piston and preferably have a diameter of 2 µm. Alternatively, the pillars can be circular with a diameter of 200 µm in order to apply a uniform compression stress to individual cells.

The piston can be made of a polymeric compound, such as polydimethylsiloxane (PDMS) in order to easily form microfabricated structures. Alternatively, the piston can be made of gel or other material suitable for the purpose of applying a compression stress to underlying cells.

According to another embodiment of the present invention, the deformable membrane has a thickness comprised between 100 µm and 140 µm, preferably 120 µm. Such a thin membrane allows a finely controlled pressure transfer from the air inside the chamber to the piston and then to the cells.

In an embodiment of the present application, the central internal cavity further comprises an upper opening aligned with the lower opening and sealable with a transparent coverslip for allowing transmitted light to pass through the upper and the lower opening.

This is extremely useful when the cell compression device is used in combination with an inverted light microscope located at the bottom of the device, for example at the bottom of the cell culture dish interlocked with the main body of the device. The kit for cell compression according to the present invention comprises a cell compression device according to any embodiment described above and a mounting tool insertable in a lower portion of the cell compression device for pre-stressing the deformable membrane before said membrane is clamped to the main body and left in the relaxed configuration. The main body comprises recess means and the mounting tool comprises projecting means to be inserted into said recess means.

The mounting tool is a critical element to first pre-stress the membrane and then relax the membrane in a suspended configuration when the mounting tool is removed.

The mounting tool can have a circular profile having an upper round edge apt to be inserted in an annulus recess in the lower portion of the main body of the cell compression device. It is appreciated that the mounting tool can further comprise an upper portion that is insertable, at least in part, into the central internal cavity of the main body when the mounting tool is inserted in the main body. Preferably, the shape of this upper portion is similar to the shape of the central internal cavity. In case the central internal cavity is cylindrical, the upper portion of the mounting tool can have a circular shape with a diameter lower that the diameter of the central internal cavity. Due to this particular configuration of the mounting tool, when the mounting tool is inserted into the lower portion of the main body, the interposed membrane, that follows the profile of the mounting tool, is partially pushed inside the central internal cavity of the main body. As a consequence, after clamping the membrane using the clamping means, the length of the membrane between two clamping points is lower than the distance between these two clamping points at the main body. This length difference corresponds to the length of the membrane inserted in the central internal cavity due to the push of the upper portion of the mounting tool. Therefore, when the mounting tool is removed, the membrane will relax in a suspended state.

In order to automatically align the piston with the center of the variable pressure chamber, the mounting tool further comprises a central recess to receive the piston attached to the membrane when said mounting tool is inserted in the lower portion of the cell compression device. In particular, the central recess is located in the upper portion of the mounting tool.

The method for applying dynamic compression stress to cells according to the present application comprises the steps of providing a main body having a central cavity with a symmetry longitudinal axis A and a lower opening aligned with said axis A and of providing a relaxed membrane having a piston attached thereto, the membrane assuming a relaxed configuration, wherein the membrane is not under tension and the piston comprising a main portion with a symmetry longitudinal axis B.

The method furthermore comprises the step of clamping the membrane to the main body at the lower opening to close the central cavity and forming a variable pressure chamber, the attached piston being located outside said variable pressure chamber and the membrane being clamped to the main body in correspondence of clamping points, wherein in an assembled configuration, when the deformable membrane is clamped to the main body, said membrane is in the relaxed configuration, the distance of two opposing clamping points being lower than the length of the membrane between said to opposing clamping points, and the symmetry longitudinal axis B of the main portion of the piston coincides with the symmetry longitudinal axis A of the central cavity.

Also, the method comprises locking a lower portion of the main body to a cell culture dish, varying the air pressure inside the variable pressure chamber and applying a dynamic compression stress to the cells in the cell culture dish based on the direct transfer of air pressure to the piston and then to the cells through the membrane in the relaxed configuration.

According to the present application, before the step of clamping the membrane to the main body, the method further comprises the step of inserting a mounting tool in a lower portion of the main body and pre-stressing the deformable membrane and wherein after the step of clamping the membrane to the main body, the method comprises the step of removing the mounting tool thereby leaving the membrane in the relaxed configuration.

In particular, the dynamic compression stress applied to single cells is at the precision of nano newtons.

Also, the lower movement of the piston in contact with cells is at the precision of microns.

It is noted that all the features characterizing the device and the kit described above also apply for the method according to the present application. Therefore, similar features are not hereby repeated.

In summary, the cell compression device, the kit and the corresponding method for applying dynamic compression stress to cells are characterized by following advantages.

The feature according to which a relaxed or /suspended membrane is used to directly transfer air pressure to cells is fundamental to achieve high precision (nano newtons) compression stress to single cells. Differently from prior art methods, the membrane is not elastically deformed/tensed and moved by liquid pressure, where the membrane tension would interfere with the amount and uniform distribution of compression force on the cells.

By using the cell compression device according to the present application, it is possible to apply ramp pressure as well as cyclic compression at nano newton precision to mimic physiological condition.

Furthermore, the piston of the cell compression device according to the present application is versatile. The piston can be made of PDMS, which provide its great potential in using microfabrication to create variety of micron sized geometry, such as micropillars for localized cell compression. The piston can also be made of gels to mimic physiological condition. The versatility of piston material as well as its potential for surface modification can provide novel applications. The piston surface can be coated with extra cellular matrix (ECM) protein to provide additional signaling to cells, such as 3D or sandwich cell culture. The ECM protein coated piston can also be used to adhere to the top of the cells followed by vertical cell stretching experiment.

In addition, the cell compression device according to the present application can be mounted on any cell culture dish so as to compress cells as well as tissue samples. It can be adapted to any microscope for high resolution imaging.

The device, the kit and the method according to the present invention are designed and configured to be utilized for different applications and under different conditions. In particular, the cell compression device is designed for high resolution, live cell as well as fixed cell immunofluorescence imaging for variety of cell experiments requiring mechanical stimuli. Specifically, to study cell morphology/cytoskeleton/nucleus transcription factor/gene expression under mechanical compression condition.

Preferred embodiments of a cell compression device, the kit and the method in accordance with the invention will be explained herein below in greater detail with reference to the accompanying drawings, in which:
- Fig. 1a-1b: show a schematic diagram of a cell compression device in assembled view (a) and in an exploded view (b);
- Fig. 2: shows a schematic view of the kit according to the present application in an exploded view;
- Fig. 3a-3d: show a schematic representation of the clamping procedure of the membrane to main body of the device according to the present application;
- Fig. 4: show a detail of the connection between the cell compression device and a cell culture dish;
- Fig. 5: show a flow diagram of the method according to the present application;

- Fig. 6a-6b: show experimental results of a step increasing compression stress on U2OS cells;
- Fig. 7a-7b: show experimental results of a cyclic compression stress on U2OS cells;
- Fig. 8a-8b: show the experimental results of applying a piston with micropillars (a) and a round piston (b); and
- Fig. 9a-9b: show additional experimental results on cell compression using the device according to the present application.

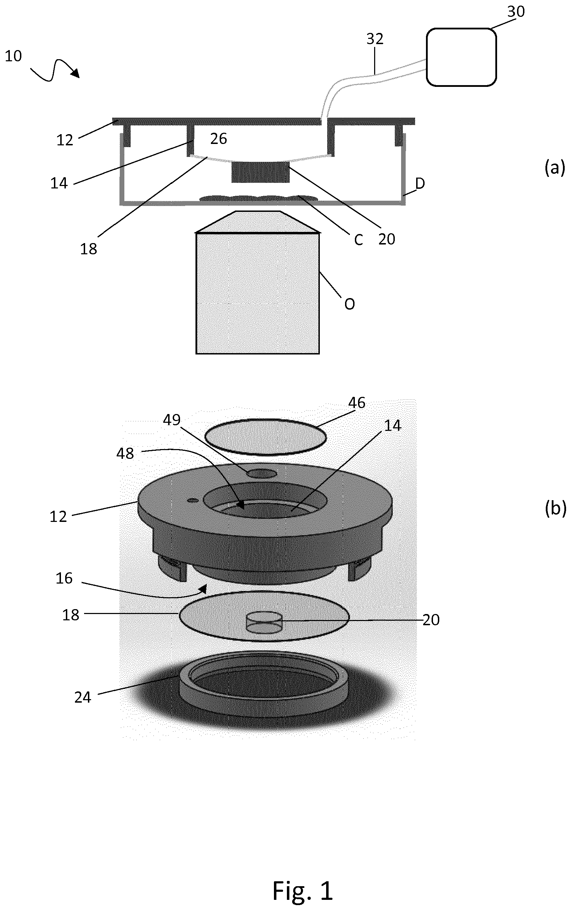

Figures 1a and 1b describe the cell compression device 10 according to a preferred embodiment of the present application. The device 10 comprises a main body 12 with a central internal cavity 14. The membrane 18 is located between the main body 12 and the clamping means 24 in the form of a clamping ring in correspondence of the lower opening 16 of the cavity 14. Once the membrane 18 is clamped to the main body 12, the central internal cavity 14 is closed, thereby forming a variable pressure chamber 26. In an assembled configuration (fig. 1a), the central internal cavity 14 is connected to a pressure regulator 30 through connecting means 32. In this way, the air pressure inside the chamber 26 can be varied and the piston can be moved in order to apply a load to the cells C distributed on the bottom of a culture cell dish D. In order to investigate the effect of the compression stress on the cells C, the objective O of an optical microscope can be located underneath the dish D in correspondence of the cells C. In order to allow the transmitted light of the microscope to pass through the chamber 26, the main body, that is the central internal cavity is provided with an upper aperture 48 that can be sealed with a transparent coverslip 46 (fig. 1b). Fig. 1b furthermore shows that the main body 12 is also provided with an additional upper opening 49 used to connect the pressure regulator means 30 to the device 10 and in particular to the central internal cavity 14.

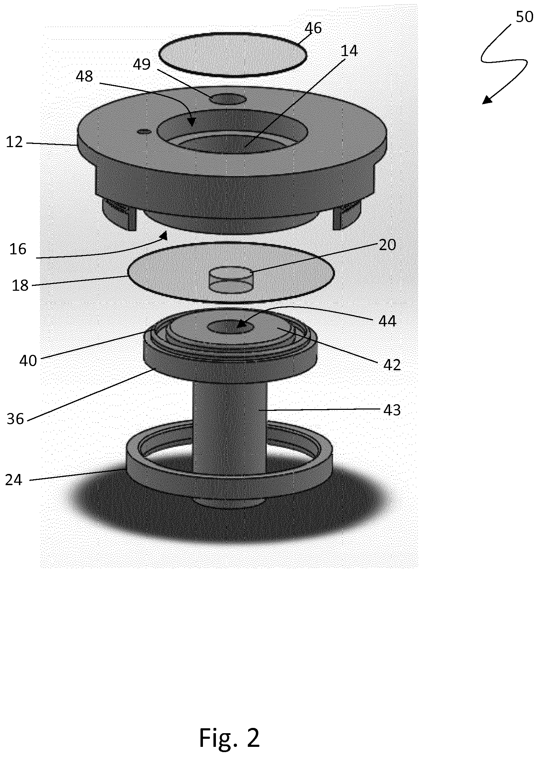

Figure 2 shows the kit 50 according to the present application in an exploded view. The kit 50 comprises the cell compression device 10 as already described in figures 1a and 1b with the addition of a mounting tool 36. The mounting tool 36 comprises an upper portion 42 and a lower portion 43 connected to the upper portion 42. In correspondence of the upper portion 42, the mounting tool 36 comprises projecting means 40 in the form of a projecting ring and a central recess 44. From figure 2 is clear that the mounting tool 36 is configured to push the membrane 18 upwards towards the central internal cavity 14 at the lower aperture 16 of the main body 12 and that central recess 44 is formed to receive the piston 20. It is noted that the mounting tool 36 has a circular form represented by the combination of a cylindrical element (lower portion 43) connected to a disc-shaped element in the upper part of the tool 36, the disc-shaped element having a greater diameter than that of the cylindrical element. Also, it is noted that the clamping means 24 has the form of a ring with a diameter greater than the maximum diameter of the mounting tool 36, that is of the disc-shaped element. In this way, it is possible to attach and/or detach the mounting tool 36 even when the clamping means 24 is already fixed to the main body 12.

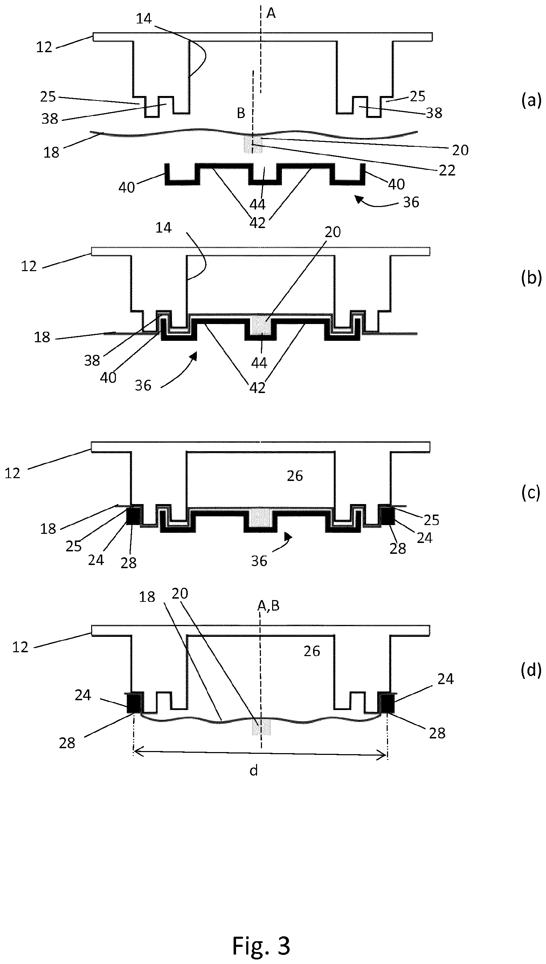

Figure 3 shows the procedure for mounting the membrane 18 to the main body 12. In particular, figure 3a shows a cross section of the main body 12 with a central internal cavity 14 having a symmetry longitudinal axis A, the deformable membrane 18 with a piston 20 attached thereto, and the mounting tool 36 in correspondence of the lower portion of the main body 12. The piston 20 has a main portion 22 with a symmetry longitudinal axis B, wherein the axis B is initially not aligned with the axis A. It is noted that the mounting tool 36 comprises at the edge projecting means 40 configured to be inserted in the corresponding recess means 38 of the main body 12. Also, the mounting tool comprises an upper portion 42 and a central cavity 44.

Figure 3b shows how the membrane 18 is pre-stressed as a consequence of the insertion of the mounting tool 36 into the lower portion of the main body 12 of the device 10. In particular, the projection means 40 of the mounting tool 36 are inserted in the recess means 38 of the main body 12. It is noted that the upper portion 42 of the mounting tool 36 is partially inserted in the central internal cavity 14 and the piston 20 is inserted in the central recess 44.

Figure 3c shows that the clamping means 24 is fixed to the main body 12 by inserting upper edges of the clamping means 24 or the clamping means 24 itself into dedicated clamping recess means 25 present in the lower portion of the main body 12. Since the membrane 18 is interposed between the clamping means 24 and the main body 12 in correspondence of clamping points 28, the membrane 18 is in turn clamped to the main body 12.

Figure 3d shows the removal of the mounting tool 36 from the main body 12. Accordingly, the membrane 18 is still clamped to the main body 12 but is left in a relaxed or suspended configuration. Form figure 3d is clear that the distance d between two opposing clamping points 28 is lower than the length of the membrane 18 between these two points. It is noted that the difference between the distance d and the membrane 18 length is due to the additional length of the membrane 18 used to fix the mounted tool 38 to the main body 12 and specifically to insert the projecting means 40 into the recess means 38 as well as the upper portion 42 into the central internal cavity 14. This is because by fixing the mounting tool 36 to the main body 12, the membrane 18 disposed therebetween follows the profile to the mounting tool 36. Furthermore, it is noted that by inserting the mounting tool 36 in the main body 12, that is by inserting the projecting means 40 into the recess means 38, the piston 20 is received by the central recess 44. In this way, it is performed an automatic alignment of the piston 20 with respect to the center of the pressure variable chamber 26 (i.e. the symmetry axes A and B coincide).

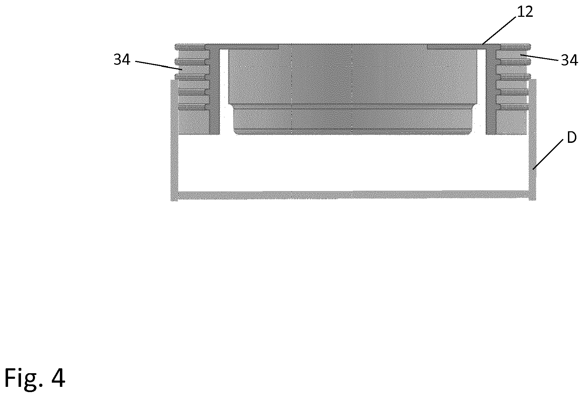

Figure 4 shows a cross section of the cell compression device 10 without the membrane 18 fixed to a cell culture dish D. It is noted that the device 10, that is the lower portion of the main body 12, is provided with connection means 34 having mechanical ribs that abut against the internal edges of a Petri dish D. In this way, the cell compression device 10 is firmly fixed relative to the dish D.

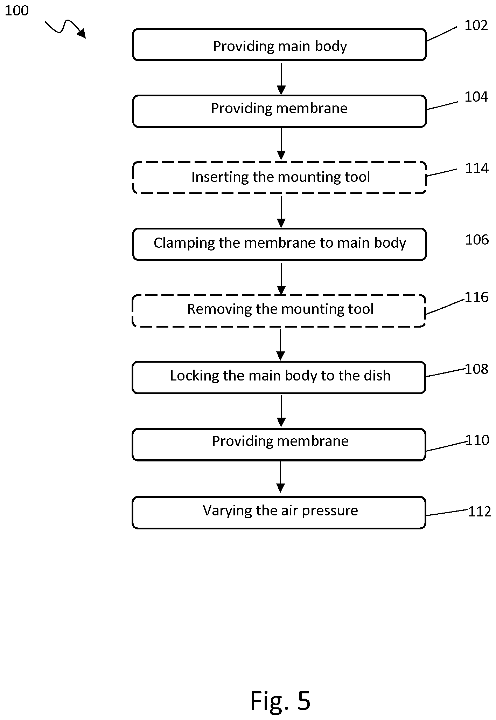

Figure 5 shows a flow chart describing the method 100 for applying dynamic compression stress to cells according to the present application.

The method 100 comprises the steps of providing 102 a main body 12, providing 104 a deformable membrane 18 having a piston 20 attached thereto, and clamping 106 the membrane 18 to the main body 12 at the lower opening 16 to close the central cavity 14 and forming a variable pressure chamber 26, wherein in the assembled configuration, the membrane 18 is in the relaxed configuration. The method 100 furthermore comprises the step of locking 108 a lower portion of the main body 12 to a cell culture dish D, varying 110 the air pressure inside the variable pressure chamber 26, and applying 112 a dynamic compression stress to the cells C in the cell culture dish D based on the direct transfer of air pressure to the piston 20 and then to the cells C through the membrane 18 in the relaxed configuration.

The method furthermore comprises the step of inserting 114 a mounting tool 36 in a lower portion of the main body 12 and pre-stressing the deformable membrane 18 before the step 106 of clamping the membrane 18 to the main body 12. Also comprises the step of removing 116 the mounting tool 36 from the main body 12 thereby leaving the membrane 18 in the relaxed configuration. It is noted that the membrane 18 can be pre-stressed in other different ways than using exactly the mounting tool 36 as described in the present application. Therefore, the method 100 can also be performed without the steps 114 and 116 assuming however that the membrane 18 is maintained in a relaxed configuration as intended in the present application after performing the clamping step 106.

### Materials and Methods

The cell compression device 10 according to the present application is designed using Solidworks, and the components are 3D printed (Form 2, Formlabs) and assembled. The cell compression device components were printed using Dental SG resin (Formlabs) for its biocompatibility. All the components were printed and then washed with IPA for 20 minutes, followed by post processing in UV chamber as suggested by Formlabs. As shown in figures 1b and 2, a 20 mm diameter coverslip 46 was glued on the top center of the cell compression device to seal the air chamber 26 and allow transmitted light of microscope to pass through. A membrane 18 of silicone attached with a piston 20 was then clamped to the bottom of the main body 12 by a clamping tool 36 as shown in figure 3. The mounting tool 36 is critical to first prestress the membrane 18 and then relax the membrane 18 to a suspended state when the mounting tool 36 is removed. Also the mounting tool 36 is important to make sure that the piston 20 is precisely located at the center of the membrane 18 and the air chamber 26. The assembled cell compression device 10 was connected to the air pressure regulator 30 and then locked to cell culture dish D through mechanical ridge 34 as shown in figure 4.

Cells C were plated on the glass bottom of a Petri dish D and maintained in cell incubator. Before any experiment, the cell compression device 10 was capped and locked on the cell culture dish 10. Cell imaging was captured using 40x oil lens (NA=1.3) in Perkin Elmer spinning disk microscope.

### RESULTS

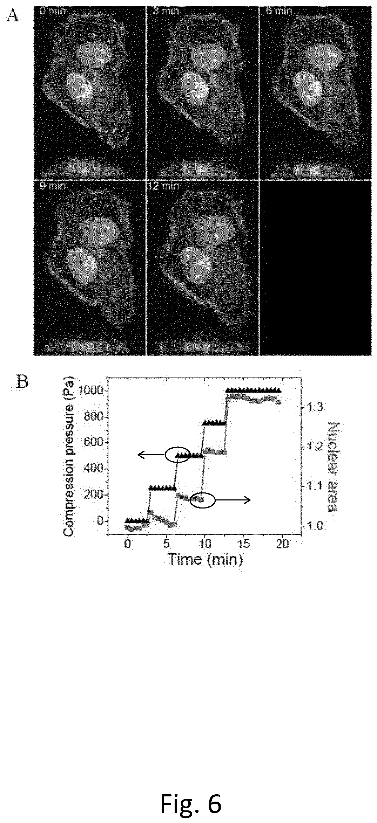

Figure 6 shows a step increasing compression on U2OS cells. Fig. 6a shows a high resolution confocal imaging with its side view of U2OS cells compressed by step increasing pressure. The nucleus was labeled by H2B-GFP, and the actin cytoskeleton was labeled by lifeact-GFP. Fig. 6b shows the plot of step increasing compression applied and its corresponding nuclear projection area change over time. The pressure increased from 0 mbar to 10 mbar in 4 steps (2.5 mbar each step)

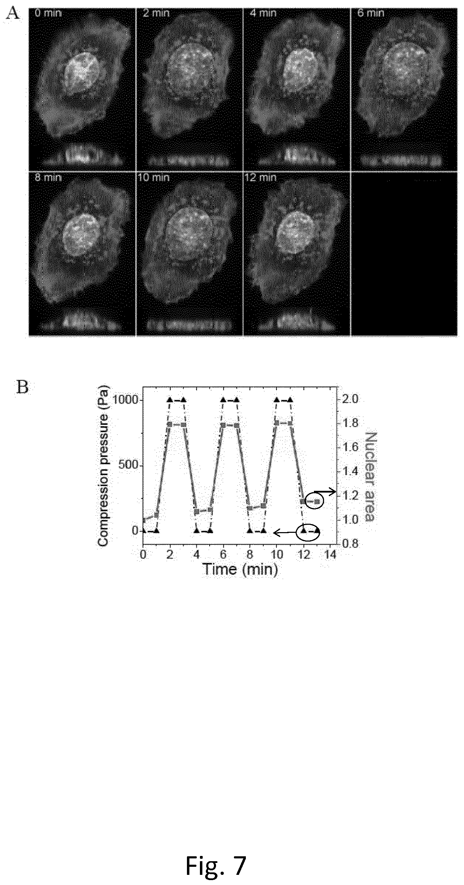

Figure 7 shows a cyclic compression on U2OS cells. Figure 7a shows a high resolution confocal imaging with its side view of U2OS cells compressed by cyclic pressure. The nucleus was labeled by H2B-GFP, and the actin cytoskeleton was labeled by lifeact-GFP. Fig. 7b shows the plot of cyclic compression applied and its corresponding nuclear projection area change over time. The compression pressure is 10 mbar.

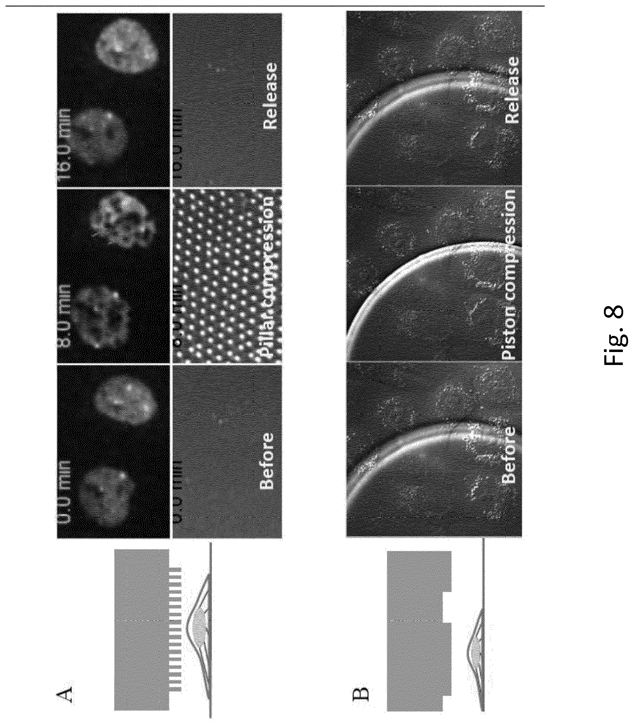

Figure 8 shows versatile compression pistons with microfabricated PDMS for variety of applications. Fig. 8a shows PDMS piston with PDMS micropillar (2 µm diameter) to apply localized compression (indentation) to U2OS nucleus. Fig. 8b shows PDMS round pistons (200 µm diameter) to apply uniform compression to individual U2OS cells.

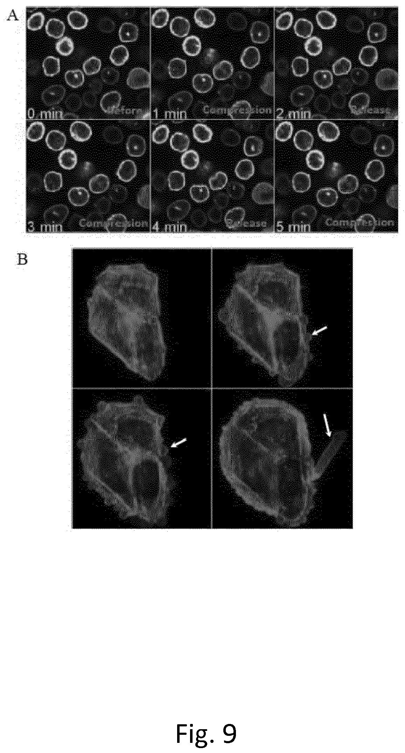

Figure 9 shows versatile applications of cell compression device. Fig. 9a shows cyclic cell compression of MCF10A cells (nucleus labeled by lamin A-GFP) remodeling of nuclear morphology. Fig. 9b shows step increasing cell compression of U2OS cells inducing formation of blobbing and its transformation to tubular blobbing (lobopodia).

It was found that performing three repeated cell compressions using the device of the invention induced actin polymerization at the perinuclear region.

## Claims

1. Cell compression device (10) for applying dynamic compression stress to cells (C), the device (10) comprising:
a main body (12) having a central internal cavity (14) with a symmetry longitudinal axis (A) and a lower opening (16) aligned with said axis (A),
a deformable membrane (18) having a piston (20) attached thereto, the piston (20) comprising a main portion (22) with a symmetry longitudinal axis (B), and
clamping means (24) to clamp the membrane (18) to the main body (12) at the lower opening (14) to close the central internal cavity (16) so that the closed central internal cavity (16) forms a variable pressure chamber (26), the attached piston (20) being located outside said variable pressure chamber (26) and the membrane (18) being clamped to the main body (12) in correspondence of clamping points (28),
**characterized in that** in an assembled configuration, when the deformable membrane (18) is clamped to the main body (12), said membrane (18) is in a relaxed configuration, wherein the distance of two opposing clamping points (28) is lower than the length of the membrane (18) between said two opposing clamping points (28), and the symmetry longitudinal axis (B) of the main portion (22) of the piston (20) coincides with the symmetry longitudinal axis (A) of the central internal cavity (14) .

2. The device (10) of claim 1, wherein the central internal cavity (14) is connectable to pressure regulator means (30) for varying the air pressure inside the variable pressure chamber (26).

3. The device (10) according to any one of the preceding claims, wherein the main body (12) further comprises connection means (34) to directly interlock the device (10) to a cell culture dish (D).

4. The device (10) according to any one of the preceding claims, wherein the piston (20) comprises a plurality of micropillars and/or
wherein the piston (20) is made of a polymeric compound such as polydimethylsiloxane or gel.

5. The device (10) according to any one of the preceding claims, wherein the deformable membrane (18) has a thickness comprised between 100 µm and 140 µm, preferably 120 µm.

6. The device (10) according to any one of the preceding claims, wherein the central internal cavity (14) further comprises an upper opening (48) aligned with the lower opening (16) and sealable with a transparent coverslip (46) for allowing transmitted light to pass through the upper and the lower opening (48, 16).

7. The device (10) according to any one of the preceding claims, configurable for high resolution, live cell as well as fixed cell immunofluorescence imaging to study cell morphology/cytoskeleton/nucleus transcription factor/gene expression under mechanical compression condition.

8. Kit (50) for cell compression comprising:
a cell compression device (10) according to any of the preceding claims, and
a mounting tool (36) insertable in a lower portion of the cell compression device (10) for pre-stressing the deformable membrane (18) before said membrane (18) is clamped to the main body (12) and left in the relaxed configuration,
wherein the main body (12) comprises recess means (38) and the mounting tool (36) comprises projecting means (40) to be inserted into said recess means (38).

9. The kit (50) according to claim 8, wherein the mounting tool (36) further comprises an upper portion (42) that is insertable, at least in part, into the central internal cavity (14) of the main body (12) when the mounting tool (36) is inserted in the main body (12).

10. The kit (50) according to claim 8 or 9, wherein the mounting tool (36) further comprises a central recess (44) to receive the piston (20) attached to the membrane (18) when said mounting tool (36) is inserted in the lower portion of the cell compression device (10).

11. The kit (50) according to any one of the claims 8 to 10, further comprising pressure regulator means (30) connectable to the central cavity (14) of the cell compression device (10) for varying the air pressure inside the variable pressure chamber (26).

12. Method (100) for applying dynamic compression stress to cells (C), the method (100) comprising:
providing (102) a main body (12) having a central cavity (14) with a symmetry longitudinal axis (A) and a lower opening (16) aligned with said axis (A),
providing (104) a deformable membrane (18) having a piston (20) attached thereto, the piston (20) comprising a main portion (22) with a symmetry longitudinal axis (B),
clamping (106) the membrane (18) to the main body (12) at the lower opening (16) to close the central cavity (14) and forming a variable pressure chamber (26), the attached piston (20) being located outside said variable pressure chamber (26) and the membrane (18) being clamped to the main body (12) in correspondence of clamping points (28), wherein in an assembled configuration when the deformable membrane (18) is clamped to the main body (12), said membrane (18) is in a relaxed configuration, wherein the distance (d) of two opposing clamping points (28) is lower than the length of the membrane (18) between said two opposing clamping points (28), and the symmetry longitudinal axis (B) of the main portion (22) of the piston (20) coincides with the symmetry longitudinal axis (A) of the central cavity (14),
locking (108) a lower portion of the main body (12) to a cell culture dish (D),
varying (110) the air pressure inside the variable pressure chamber (26),
applying (112) a dynamic compression stress to the cells (C) in the cell culture dish (D) based on the direct transfer of air pressure to the piston (20) and then to the cells (C) through the membrane (18) in the relaxed configuration.

13. The method (100) according to claim 12, wherein before the step of clamping (106) the membrane (18) to the main body (12), the method (100) further comprises the step of inserting (114) a mounting tool (36) in a lower portion of the main body (12) and pre-stressing the deformable membrane (18) and wherein after the step of clamping (106) the membrane (18) to the main body (12), the method (100) comprises the step of removing (116) the mounting tool (36), thereby leaving the membrane (18) in the relaxed configuration.

14. The method (100) according to claim 12 or 13, wherein the dynamic compression stress applied to the single cells (C) is in the precision of nano newtons.

15. The method (100) according to any one of claims 12 to 14, wherein the step of varying (110) the air pressure inside the variable pressure chamber (26) determines a lower movement of the piston at the precision of microns.

## Patentansprüche

1. Zellkompressionsvorrichtung (10) zum Ausüben einer dynamischen Kompressionsspannung auf Zellen (C), wobei die Vorrichtung (10) umfasst:
einen Hauptkörper (12), der einen zentralen inneren Hohlraum (14) mit einer Symmetrie-Längsachse (A) und eine auf die Achse (A) ausgerichtete untere Öffnung (16) aufweist,
eine verformbare Membran (18), die einen daran angebrachten Kolben (20) aufweist, wobei der Kolben (20) einen Hauptabschnitt (22) mit einer Symmetrie-Längsachse (B) umfasst, und
Klemmmittel (24), um die Membran (18) an der unteren Öffnung (14) an den Hauptkörper (12) zu klemmen, um den zentralen inneren Hohlraum (16) zu verschließen, sodass der geschlossene zentrale innere Hohlraum (16) eine Kammer (26) mit veränderlichem Druck bildet, wobei der angebrachte Kolben (20) außerhalb der Kammer (26) mit veränderlichem Druck angeordnet ist und die Membran (18) in Übereinstimmung mit Klemmpunkten (28) an den Hauptkörper (12) geklemmt ist,
**dadurch gekennzeichnet, dass** in einer zusammengebauten Konfiguration, wenn die verformbare Membran (18) an den Hauptkörper (12) geklemmt ist, die Membran (18) in einer entspannten Konfiguration ist, wobei der Abstand zweier gegenüberliegender Klemmpunkte (28) geringer als die Länge der Membran (18) zwischen den zwei gegenüberliegenden Klemmpunkten (28) ist, und die Symmetrie-Längsachse (B) des Hauptabschnitts (22) des Kolbens (20) mit der Symmetrie-Längsachse (A) des zentralen inneren Hohlraums (14) zusammenfällt.

2. Vorrichtung (10) nach Anspruch 1, wobei der zentrale innere Hohlraum (14) mit Druckregelungsmitteln (30) zum Verändern des Luftdrucks im Inneren der Kammer (26) mit veränderlichem Druck verbunden werden kann.

3. Vorrichtung (10) nach einem der vorhergehenden Ansprüche, wobei der Hauptkörper (12) ferner Verbindungsmittel (34) umfasst, um die Vorrichtung (10) direkt mit einer Zellkulturschale (D) zu verriegeln.

4. Vorrichtung (10) nach einem der vorhergehenden Ansprüche, wobei der Kolben (20) eine Vielzahl von Mikrosäulen umfasst und/oder
wobei der Kolben (20) aus einer polymeren Verbindung wie Polydimethylsiloxan oder Gel hergestellt ist.

5. Vorrichtung (10) nach einem der vorhergehenden Ansprüche, wobei die verformbare Membran (18) eine Dicke zwischen 100 µm und 140 µm, vorzugsweise von 120 µm, aufweist.

6. Vorrichtung (10) nach einem der vorhergehenden Ansprüche, wobei der zentrale innere Hohlraum (14) ferner eine obere Öffnung (48) aufweist, die auf die untere Öffnung (16) ausgerichtet ist und mit einem transparenten Deckglas (46) verschließbar ist, um zu ermöglichen, dass durchfallendes Licht durch die obere und die untere Öffnung (48, 16) hindurchgeht.

7. Vorrichtung (10) nach einem der vorhergehenden Ansprüche, konfigurierbar für hochauflösende Immunfluoreszenz-Bildgebung von lebenden Zellen sowie fixierten Zellen zur Untersuchung von Zellmorphologie/Zytoskelett/Kern-Transkriptionsfaktor/Genexpression unter mechanischen Kompressionsbedingungen.

8. Kit (50) zur Zellkompression, umfassend:
eine Zellkompressionsvorrichtung (10) nach einem der vorhergehenden Ansprüche, und
ein Montagewerkzeug (36), das in einen unteren Abschnitt der Zellkompressionsvorrichtung (10) eingesetzt werden kann, um die verformbare Membran (18) vorzuspannen, bevor die Membran (18) an den Hauptkörper (12) geklemmt wird und in der entspannten Konfiguration belassen wird,
wobei der Hauptkörper (12) Ausnehmungsmittel (38) umfasst und das Montagewerkzeug (36) Vorsprungmittel (40) zum Einsetzen in die Ausnehmungsmittel (38) umfasst.

9. Kit (50) nach Anspruch 8, wobei das Montagewerkzeug (36) ferner einen oberen Abschnitt (42) umfasst, der wenigstens teilweise in den zentralen inneren Hohlraum (14) des Hauptkörpers (12) eingesetzt werden kann, wenn das Montagewerkzeug (36) in den Hauptkörper (12) eingesetzt ist.

10. Kit (50) nach Anspruch 8 oder 9, wobei das Montagewerkzeug (36) ferner eine zentrale Ausnehmung (44) umfasst, um den an der Membran (18) angebrachten Kolben (20) aufzunehmen, wenn das Montagewerkzeug (36) in den unteren Abschnitt der Zellkompressionsvorrichtung (10) eingesetzt ist.

11. Kit (50) nach einem der Ansprüche 8 bis 10, ferner umfassend Druckregelungsmittel (30), die mit dem zentralen Hohlraum (14) der Zellkompressionsvorrichtung (10) verbunden werden kann, um den Luftdruck im Inneren der Kammer (26) mit veränderlichem Druck zu verändern.

12. Verfahren (100) zum Ausüben einer dynamischen Kompressionsspannung an Zellen (C), wobei das Verfahren (100) umfasst:
Bereitstellen (102) eines Hauptkörpers (12), der einen zentralen Hohlraum (14) mit einer Symmetrie-Längsachse (A) und eine auf die Achse (A) ausgerichtete untere Öffnung (16) aufweist,
Bereitstellen (104) einer verformbaren Membran (18), die einen daran angebrachten Kolben (20) aufweist, wobei der Kolben (20) einen Hauptabschnitt (22) mit einer Symmetrie-Längsachse (B) umfasst,
Klemmen (106) der Membran (18) an den Hauptkörper (12) an der unteren Öffnung (16), um den zentralen Hohlraum (14) zu verschließen und eine Kammer (26) mit veränderlichem Druck zu bilden, wobei der angebrachte Kolben (20) außerhalb der Kammer (26) mit veränderlichem Druck angeordnet ist und die Membran (18) in Übereinstimmung mit Klemmpunkten (28) an den Hauptkörper (12) geklemmt ist, wobei in einer zusammengebauten Konfiguration, wenn die verformbare Membran (18) an den Hauptkörper (12) geklemmt ist, die Membran (18) in einer entspannten Konfiguration ist, wobei der Abstand (d) zweier gegenüberliegender Klemmpunkte (28) geringer als die Länge der Membran (18) zwischen den zwei gegenüberliegenden Klemmpunkten (28) ist und die Symmetrie-Längsachse (B) des Hauptabschnitts (22) des Kolbens (20) mit der Symmetrie-Längsachse (A) des zentralen Hohlraums (14) zusammenfällt,
Verriegeln (108) eines unteren Abschnitts des Hauptkörpers (12) an einer Zellkulturschale (D),
Verändern (110) des Luftdrucks im Inneren der Kammer (26) mit veränderlichem Druck,
Ausüben (112) einer dynamischen Kompressionsspannung auf die Zellen (C) in der Zellkulturschale (D) auf der Grundlage der direkten Übertragung von Luftdruck auf den Kolben (20) und anschließend auf die Zellen (C) durch die Membran (18) in der entspannten Konfiguration.

13. Verfahren (100) nach Anspruch 12, wobei vor dem Schritt des Klemmens (106) der Membran (18) an den Hauptkörper (12) das Verfahren (100) ferner den Schritt des Einsetzens (114) eines Montagewerkzeugs (36) in einen unteren Abschnitt des Hauptkörpers (12) und des Vorspannens der verformbaren Membran (18) umfasst und wobei nach dem Schritt des Klemmens (106) der Membran (18) an den Hauptkörper (12) das Verfahren (100) den Schritt des Entfernens (116) des Montagewerkzeugs (36) umfasst, wodurch die Membran (18) in der entspannten Konfiguration belassen wird.

14. Verfahren (100) nach Anspruch 12 oder 13, wobei die dynamische Druckspannung, die auf die einzelnen Zellen (C) ausgeübt wird, die Genauigkeit von Nano-Newton aufweist.

15. Verfahren (100) nach einem der Ansprüche 12 bis 14, wobei der Schritt des Veränderns (110) des Luftdrucks im Inneren Kammer (26) mit veränderlichem Druck eine geringere Bewegung des Kolbens mit der Genauigkeit von Mikrometern bestimmt.

## Revendications

1. Dispositif de compression de cellules (10) pour appliquer une contrainte de compression dynamique à des cellules (C), le dispositif (10) comprenant :
un corps principal (12) ayant une cavité interne centrale (14) avec un axe longitudinal de symétrie (A) et une ouverture inférieure (16) alignée sur ledit axe (A),
une membrane déformable (18) à laquelle un piston (20) est fixé, le piston (20) comprenant une partie principale (22) avec un axe longitudinal de symétrie (B), et
des moyens de serrage (24) pour serrer la membrane (18) sur le corps principal (12) au niveau de l'ouverture inférieure (14) pour fermer la cavité interne centrale (16) de manière à ce que la cavité interne centrale (16) fermée forme une chambre à pression variable (26), le piston fixé (20) étant situé à l'extérieur de ladite chambre à pression variable (26) et la membrane (18) étant serrée sur le corps principal (12) en correspondance des points de serrage (28),
**caractérisé en ce que** dans une configuration assemblée, lorsque la membrane déformable (18) est serrée sur le corps principal (12), ladite membrane (18) se trouve dans une configuration relâchée, dans laquelle la distance entre deux points de serrage opposés (28) est inférieure à la longueur de la membrane (18) entre lesdits deux points de serrage opposés (28), et l'axe longitudinal de symétrie (B) de la partie principale (22) du piston (20) coïncide avec l'axe longitudinal de symétrie (A) de la cavité interne centrale (14).

2. Dispositif (10) selon la revendication 1, dans lequel la cavité interne centrale (14) peut être raccordée à un moyen de régulation de pression (30) pour faire varier la pression d'air à l'intérieur de la chambre à pression variable (26).

3. Dispositif (10) selon l'une quelconque des revendications précédentes, dans lequel le corps principal (12) comprend en outre un moyen de raccordement (34) pour directement enclencher le dispositif (10) sur une boîte de culture cellulaire (D).

4. Dispositif (10) selon l'une quelconque des revendications précédentes, dans lequel le piston (20) comprend une pluralité de micro-piliers et/ou
dans lequel le piston (20) est formé en un composé polymère tel qu'un polydiméthylsiloxane ou un gel.

5. Dispositif (10) selon l'une quelconque des revendications précédentes, dans lequel la membrane déformable (18) a une épaisseur comprise entre 100 µm et 140 µm, de préférence de 120 µm.

6. Dispositif (10) selon l'une quelconque des revendications précédentes, dans lequel la cavité interne centrale (14) comprend en outre une ouverture supérieure (48) alignée sur l'ouverture inférieure (16) et scellable avec une lamelle couvre-objet transparente (46) pour permettre le passage de la lumière transmise à travers les ouvertures supérieure et inférieure (48, 16) .

7. Dispositif (10) selon l'une quelconque des revendications précédentes, pouvant être configuré pour une imagerie par fluorescence haute résolution de cellules vivantes et de cellules fixées, pour étudier la morphologie des cellules/le cytosquelette/le facteur de transcription nucléaire/l'expression génique dans des conditions de compression mécanique.

8. Kit (50) de compression de cellules comprenant :
un dispositif de compression de cellules (10) selon l'une quelconque des revendications précédentes, et
un outil de montage (36) pouvant être inséré dans une partie inférieure du dispositif de compression de cellules (10) pour précontraindre la membrane déformable (18) avant que ladite membrane (18) ne soit serrée sur le corps principal (12) et laissée dans la configuration relâchée,
dans lequel le corps principal (12) comprend un moyen formant cavité (38) et l'outil de montage (36) comprend un moyen formant saillie (40) à insérer dans ledit moyen formant cavité (38).

9. Kit (50) selon la revendication 8, dans lequel l'outil de montage (36) comprend en outre une partie supérieure (42) qui peut être insérée, au moins en partie, dans la cavité interne centrale (14) du corps principal (12) lorsque l'outil de montage (36) est inséré dans le corps principal (12).

10. Kit (50) selon la revendication 8 ou 9, dans lequel l'outil de montage (36) comprend en outre une cavité centrale (44) pour recevoir le piston (20) fixé à la membrane (18) lorsque ledit outil de montage (36) est inséré dans la partie inférieure du dispositif de compression de cellules (10).

11. Kit (50) selon l'une quelconque des revendications 8 à 10, comprenant en outre un moyen de régulation de pression (30) pouvant être raccordé à la cavité centrale (14) du dispositif de compression de cellules (10) pour faire varier la pression d'air à l'intérieur de la chambre à pression variable (26).

12. Procédé (100) d'application d'une contrainte de compression dynamique aux cellules (C), le procédé (100) comprenant :
la fourniture (102) d'un corps principal (12) ayant une cavité centrale (14) avec un axe longitudinal de symétrie (A) et une ouverture inférieure (16), alignée avec ledit axe (A),
la fourniture (104) d'une membrane déformable (18) ayant un piston (20) fixé à celui-ci, le piston (20) comprenant une partie principale (22) avec un axe longitudinal de symétrie (B),
le serrage (106) de la membrane (18) sur le corps principal (12) au niveau de l'ouverture inférieure (16) pour fermer la cavité centrale (14) et la formation d'une chambre à pression variable (26), le piston fixé (20) étant situé à l'extérieur de ladite chambre à pression variable (26) et la membrane (18) étant serrée sur le corps principal (12) en correspondance des points de serrage (28), dans lequel dans une configuration assemblée lorsque la membrane déformable (18) est serrée au corps principal (12), ladite membrane (18) se trouve dans une configuration relâchée, dans laquelle la distance (d) entre deux points de serrage opposés (28) est inférieure à la longueur de la membrane (18) entre lesdits deux points de serrage opposés (28), et l'axe longitudinal de symétrie (B) de la partie principale (22) du piston (20) coïncide avec l'axe longitudinal de symétrie (A) de la cavité centrale (14), le verrouillage (108) d'une partie inférieure du corps principal (12) sur une boîte de culture cellulaire (D),
la variation (110) de la pression d'air à l'intérieur de la chambre à pression variable (26),
l'application (112) d'une contrainte de compression dynamique aux cellules (C) dans la boîte de culture cellulaire (D) sur la base du transfert direct de pression d'air au piston (20) puis aux cellules (C) à travers la membrane (18) dans la configuration relâchée.

13. Procédé (100) selon la revendication 12, dans lequel avant l'étape de serrage (106) de la membrane (18) sur le corps principal (12), le procédé (100) comprend en outre l'étape d'insertion (114) d'un outil de montage (36) dans une partie inférieure du corps principal (12) et de précontrainte de la membrane déformable (18) et dans lequel après l'étape de serrage (106) de la membrane (18) au corps principal (12), le procédé (100) comprend l'étape de retrait (116) de l'outil de montage (36), laissant ainsi la membrane (18) dans la configuration relâchée.

14. Procédé (100) selon la revendication 12 ou 13, dans lequel la précision de contrainte de compression dynamique appliquée aux cellules seules (C) est de l'ordre des nanonewtons.

15. Procédé (100) selon l'une quelconque des revendications 12 à 14, dans lequel l'étape de variation (110) de la pression d'air à l'intérieur de la chambre à pression variable (26) détermine un mouvement inférieur du piston à la précision de l'ordre des microns.
